# EUROPÄISCHE PATENTSCHRIFT

(11) **EP 0 841 069 B1**
(45) Veröffentlichungstag und Bekanntmachung des Hinweises auf die Patenterteilung: **02.07.2003**
(21) Anmeldenummer: 97119576.3
(22) Anmeldetag: 08.11.1997
(51) Int. Cl.: A61L 2/26, G01N 7/00, B01D 5/00

(54) **Verfahren und Vorrichtung zur Analyse von Dampf aus einer Dampfsterilisations-oder Dampfdesinfektionsanlage**
Method and apparatus for the analysis of vapour from a vapour sterilisation or disinfection plant
Méthode et dispositif pour l'analyse de la vapeur d'un appareil de stérilisation ou de désinfection de vapeur

(30) Priorität: 09.11.1996 DE 19646301
(43) Veröffentlichungstag der Anmeldung: 13.05.1998
(73) Patentinhaber: F. & M. Lautenschläger, 50972 Köln (DE)
(72) Erfinder: Weber, Wolfgang, 53842 Troisdorf-Spich (DE)
(74) Vertreter: WALTHER, WALTHER & HINZ Patentanwälte

(56) Entgegenhaltungen:
- DD-A- 262 484
- DE-A- 3 636 716
- DE-A- 4 319 402
- US-A- 3 967 494
- US-A- 4 115 068
- US-A- 4 594 223

## Beschreibung

Die vorliegende Erfindung betrifft ein Verfahren und eine Vorrichtung zur Analyse von Dampf aus einer Dampfsterilisations- oder Dampfdesinfektionsanlage gemäß den Oberbegriffen der Ansprüche 1 und 4.

Bei der Sterilisation oder Desinfektion von medizinischen Produkten, von im Krankenhaus eingesetzten Gegenständen oder von Lebensmitteln, ist es zur vollständigen Keimtötung erforderlich, einen Sattdampf hoher Reinheit zur Verfügung zu haben, denn nur ein hochreiner Sattdampf kann die zur Sterilisation bzw. Desinfektion erforderliche hohe Energie speichern. In dem zur Sterilisation bzw. Desinfektion verwendeten Speisewasser sind naturgemäß verschiedene Inertgase gebunden, die bei der Sterilisationstemperatur von 134°C und beim vorgesehenen Sterilisationsdruck nicht kondensieren und folglich im gasförmigen Zustand verbleiben. Diese gasförmig verbleibenden Gase, wie z. B. Stickstoff, Kohlendioxid, Sauerstoff usw. nehmen ein entsprechendes Volumen ein, transportieren aber im Vergleich zum Sattdampf sehr viel weniger Energie, so daß angestrebt wird, den Anteil an Inertgasen im Sattdampf möglichst gering zu halten. Zur Gewährleistung einer ordnungsgemäßen Sterilisation bzw. Desinfektion sollte ein vorgegebener Anteil an Inertgasen im Sattdampf nicht überschritten werden.

Aus der US 3,967,494 ist eine Vorrichtung zur Bestimmung der Restluft in Sterilisationsanlagen offenbart, die nach dem Gravitationsverfahren arbeitet. Dabei wird die spezifisch schwerere Luft aus der Druckkammer nach unten heraus in eine Messvorrichtung gedrückt, der Sterilisationsdampf entspannt sich und kondensiert in der Messvorrichtung aus. Es versteht sich, dass dabei lediglich die Wasseranteile auskondensieren und die im Sterilisationsdampf befindliche Luft gasförmig verbleibt. Diese nach dem Kondensieren gasförmig verbleibende Luft wird im Messgerät gesammelt und sobald die hierdurch entstandene Luftblase eine gewisse Größe erreicht hat, wird diese aus der Messvorrichtung abgelassen, so dass sich anschließend eine neue Luftblase bilden kann. Dabei wird die Zeit zwischen zwei Luftblasen gemessen. Liegt die Zeit zwischen zwei Luftblasen über einem Grenzwert, so gilt die Druckkammer als luftfrei und der eigentliche Sterilisationsprozess kann beginnen.

Beim Gravitationsverfahren kann lediglich die im Sterilisationsdampf befindliche Luft gemessen werden, nicht jedoch andere, für den Sterilisationsprozess schädliche Inertgase, da einige dieser Inertgase leichter als Wasserdampf sind und nicht auf den Boden der Druckkammer absinken. Auch können mit dieser Methode nur bedingt etwaige Lecks der Druckkammer ermittelt werden, da unbeabsichtigt eindringende Luft eine gewisse Zeit benötigt, bis sie auf den Boden der Druckkammer abgesunken ist und in das Messverfahren einbezogen werden kann. Außerdem endet die Überprüfung des Sterilisationsdampfes mit Beginn des Sterilisationsprozesses, so dass zu diesem Zeitpunkt einströmende Fremdgase oder sich erst jetzt auf den Grund der Druckkammer absetzende Fremdgase messtechnisch nicht erfasst werden.

Zur kontinuierlichen Überprüfung der Reinheit des zur Sterilisation bzw. Desinfektion verwendeten Sattdampfes sind bisher zwei Verfahren und dazugehörige Vorrichtungen bekannt. Das eine ist aus der DE 36 36 716 A 1, das andere aus der DE 43 19 402 A 1 bekannt.

Beim Verfahren nach der DE 36 36 716 A 1 wird über ein Probenröhrchen und ein Entnahmeventil eine gewisse Menge des zur Sterilisation bzw. Desinfektion bestimmten Dampfes abgepumpt und zur Kondensation in einen Luftkühler geleitet. Nachdem der Sattdampf auskondensiert ist, wird er in einem Blasenbildner beruhigt, so daß sich die im Sattdampf befindlichen Inertgase zu einzelnen Blasen sammeln können. Anschließend wird diese Probe in eine dünne Meßleitung gepumpt und mit konstanter Geschwindigkeit an einer Lichtschranke vorbeigeführt. Mit Hilfe der Lichtschranke werden dann die Zeiten gemessen, während der Kondensat und während der Blasen an der Lichtschranke vorbeiziehen. Gleichzeitig wird noch die Temperatur gemessen, so daß über die gemessene Temperatur und das Verhältnis der Blasen zum Kondensat der Anteil der Inertgase am Sattdampf ermittelt werden kann.

Bei diesem Verfahren ist es zur Erzielung exakter Meßergebnisse notwendig, ein Meßröhrchen mit einem sehr präzisen und über die gesamte Länge konstanten Innendurchmesser zur Verfügung zu haben und außerdem ist es notwendig, eine Pumpe zur Verfügung zu haben, die die auskondensierte Probe des Sattdampfes mit konstanter Geschwindigkeit an der Lichtschranke vorbeiführt. Derartige Bauteile sind sehr teuer, zumal zur Bestimmung des Inertgasanteils noch eine elektronische Recheneinheit erforderlich ist. Desweiteren ist das Meßröhrchen sehr schmutzempfindlich, was hohe Reinigungskosten zur Folge hat. Auch ist es schwer, die Vapocontrol-Vorrichtung zu justieren und zu kontrollieren. Zur Lösung der vorgenannten Schwierigkeiten wird in der DE 43 19 402 A 1 ein Verfahren zur kontinuierlichen Bestimmung des Inertgasanteils im Sattdampf vorgeschlagen. Bei diesem Verfahren wird eine aus der Dampfleitung abgezweigte und auskondensierte Probe in ein Steigrohr geleitet. Der vorhandene statische Druck dieser Probe wird über hochsensible Druckaufnehmer gemessen und entweder mit einem gespeicherten Vergleichswert oder mit einem in einem parallelen Vergleichsrohr ermittelten Vergleichswert verglichen. Dabei ist der Durchmesser des Steigrohres so gewählt, daß die Blasen aufgrund der Kapillarität nicht entweichen können, während der Durchmesser des Vergleichsrohres so groß gewählt ist, daß etwaige auftretende, aus inertgas gebildete Blasen sofort aufsteigen und das Vergleichsrohr verlassen. Über einen Vergleich des gemessenen Druckes im Steigrohr mit dem Vergleichswert wird dann der Anteil des Inertgases im Sattdampf bestimmt. Bei diesem Verfahren sind teure Ventile, teure Druckaufnehmer und teure Auswerteeinheiten erforderlich.

Beide oben genannten Verfahren beziehen ihre Probe aus der Dampfleitung und bestimmen den Anteil an Inertgasen im noch in die Sterilisations- bzw. Desinfektionskammer einzuspeisenden Sattdampf. In der Praxis kommt es jedoch immer wieder vor, daß sich in der Sterilisations- bzw. Desinfektionskammer weitere Inertgase befinden, die über eine Leckage oder mangelhafte Entlüftung in die Kammer gelangt sind. Diese Inertgase beeinträchtigen den Sterilisations- bzw. Desinfektionsprozeß genauso negativ, wie bereits im Speisewasser befindliche Inertgase. Die aus dem Stand der Technik bekannten Verfahren sind nicht in der Lage, etwaige Leckagen oder mangelhafte Entlüftung der Sterilisations- bzw. Desinfektionskammer zu erkennen und zu melden.

Davon ausgehend liegt der vorliegenden-Erfindung die Aufgabe zugrunde, ein Verfahren und eine Vorrichtung zur Analyse von Dampf aus Dampfsterilisations- oder Dampfdesinfektionsanlagen zu schaffen, welches kostengünstig realisierbar ist und welches gleichzeitig die im Speisewasser befindlichen und die in der Sterilisations- bzw. Desinfektionskammer befindlichen Inertgase erfaßt und somit gegebenenfalls vorliegende Leckagen oder mangelhafte Entlüftung erkennt und meldet.

Als technische Lösung dieser Aufgabe wird erfindungsgemäß ein Verfahren mit den Merkmalen des Anspruchs 1 und eine Vorrichtung mit den Merkmalen des Anspruchs 4 vorgeschlagen.

Das erfindungsgemäße Verfahren und die erfindungsgemäße Meßvorrichtung haben den Vorteil, daß durch die Verbindung des Meßbehälters mit der Druckkammer der Sterilisations- bzw. Desinfektionsanlage der Meßbehälter analog zur Druckkammer evakuiert bzw. mit Sattdampf gefüllt wird, so daß eine Echtzeitmessung der in der Druckkammer herrschenden Verhältnisse geschaffen ist. Hierdurch wird der tatsächlich am Sterilisier- bzw. Desinfiziergut anliegende Sattdampf gemessen, so daß auch durch etwaige Leckagen oder mangelhafte Entlüftung eintretende Gase, die sich mit dem eintretenden Sattdampf vermischen, durch die Messung erfaßt werden.

Ein weiterer Vorteil besteht darin, daß die erfindungsgemäße Vorrichtung keinerlei Pumpen, Ventile oder sonstige beweglichen Teilen benötigt, die kostenaufwendig und verschleißanfällig sind. Auch der eingesetzte Meßbehälter, die notwendige Dampfzuführungsleitung und die verwendeten Lichtschranken sind Standardbauteile und somit kostengünstig. Die erfindungsgemäße Meßvorrichtung wird einmal auf die Größe der Druckkammer ausgelegt und braucht anschließend nie mehr justiert bzw. kalibriert zu werden, so daß sich die Wartungs- und Folgekosten der erfindungsgemäßen Vorrichtung auf einen Minimum reduzieren. Durch die geschickte Ausnutzung der Kapillarität ist die Oberflächenspannung des im Meßbehälters angesammelten Kondensats so groß, daß dieses im Meßbehälter verbleibt. Gleichzeitig kann solange weiterer Sattdampf in den Meßbehälter hineingedrückt werden, bis dieser gefüllt ist, das heißt, bis der sich in der Gasblase ausbildende Druck dem Druck in der Druckkammer entspricht.

Bei einer Leckage dringt Außenluft vornehmlich während der Steigephase in die Druckkammer ein, so daß ein weiterer Vorteil darin besteht, daß der Inhalt der Druckkammer während der Steigephase überprüft wird. Hierdurch können die eingedrungenen Inertgase sofort erfaßt werden, und die Leckage kann umgehend gemeldet und ausgewertet werden.

Weitere Vorteile des erfindungsgemäßen Verfahrens und der erfindungsgemäßen Vorrichtung ergeben sich aus der Beschreibung und aus der beigefügten Zeichnung. In der Zeichnung ist ein Ausführungsbeispiel einer erfindungsgemäßen Vorrichtung dargestellt, anhand dessen die Erfindung näher erläutert wird. Dabei zeigt:
- Figur 1: ein Druck-Zeit Diagramm, in dem der Druckverlauf während des Sterilisationsprozesses dargestellt ist;
- Figur 2: eine schematische Skizze einer Sterilisationsanlage mit einem Ausführungsbeispiel einer erfindungsgemäßen Meßvorrichtung;
- Figur 3: eine geschnitten dargestellte Frontansicht der Meßvorrichtung gemäß Figur 2;
- Figur 4: eine geschnitten dargestellte Seitenansicht eines Teils der Meßvorrichtung gemäß Figur 2.

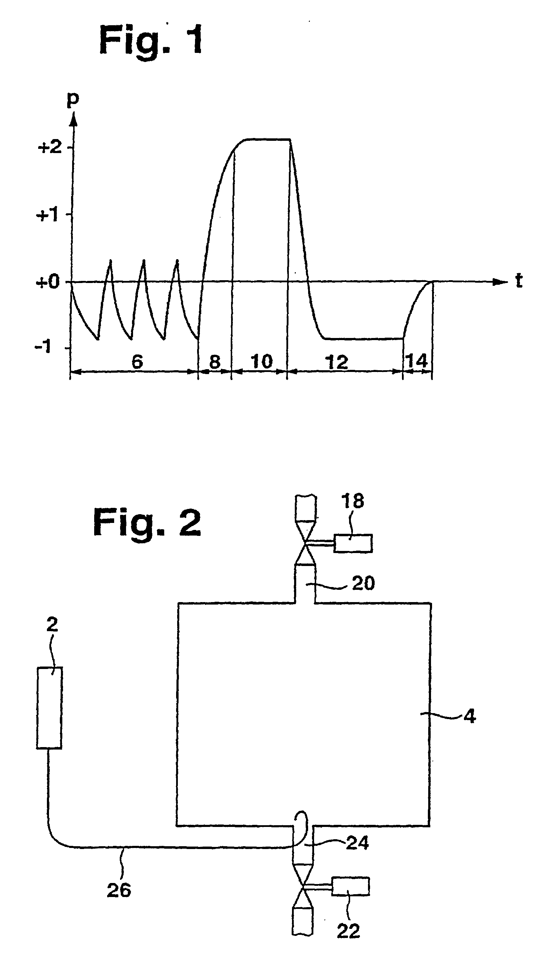

In den Figuren 2 bis 4 ist eine Dampfsterilisationsanlage mit einer erfindungsgemäßen Meßvorrichtung 2 dargestellt. Analog hierzu kann die erfindungsgemäße Meßvorrichtung 2 auch in einer Dampfdesinfektionsanlage eingesetzt werden.

Das in Figur 1 dargestellte Druck-Zeit Diagramm zeigt den Druckverlauf während des Sterilisationsprozesses. Hieraus erkennt man, daß die Druckkammer 4 der Sterilisationsanlage während der Entlüftungsphase 6 durch mehrfaches, sogenanntes Fraktionieren, nahezu vollständig evakuiert wird. Nach der Entlüftungsphase 6 folgt die Steigephase 8, in der die nunmehr vollständig evakuierte Druckkammer solange mit Sattdampf befüllt wird, bis der Sterilisationsdruck erreicht ist. Während der sogenannten Plateauphase, die bei einem normalen Sterilisationsprozeß zirka 5 Minuten dauert, wird der Sterilisationsdruck konstant gehalten. Danach folgt die Trocknungsphase 12, in der die Druckkammer wieder evakuiert wird, um das nun sterilisierte Sterilisiergut von auskondensiertem Sattdampf zu trocknen. In der anschließenden Belüftungsphase 14 wird die Druckkammer auf Umgebungsdruck gebracht, so daß die Druckkammertür vom Bedienpersonal geöffnet werden kann, um das sterilisierte Gut herauszunehmen.

In Figur 2 ist eine Sterilisationsanlage grob schematisch dargestellt. Diese Sterilisationsanlage umfaßt eine Druckkammer 4, in die eine mit einem Dampfventil 18 versehene Sattdampfzuführleitung 20 mündet. Gegenüber der Sattdampfzuführleitung 20 ist eine ein Vakuumventil 22 aufweisende Evakuierungsleitung 24 angeordnet.

An einer beliebigen Stelle der Druckkammer 4 ist eine Dampfzuführleitung 26 eingelassen, die in die Meßvorrichtung 2 mündet. Da weder in der Meßvorrichtung 2 noch in der Dampfzuführleitung 26 eine Absperrung oder ein Ventil vorgesehen ist, ist die Meßvorrichtung 2 derart mit der Druckkammer verbunden, daß deren Hohlräume miteinander kommunizieren können. Das heißt in der Meßeinrichtung 2 und der Druckkammer 4 herrscht immer derselbe Druck.

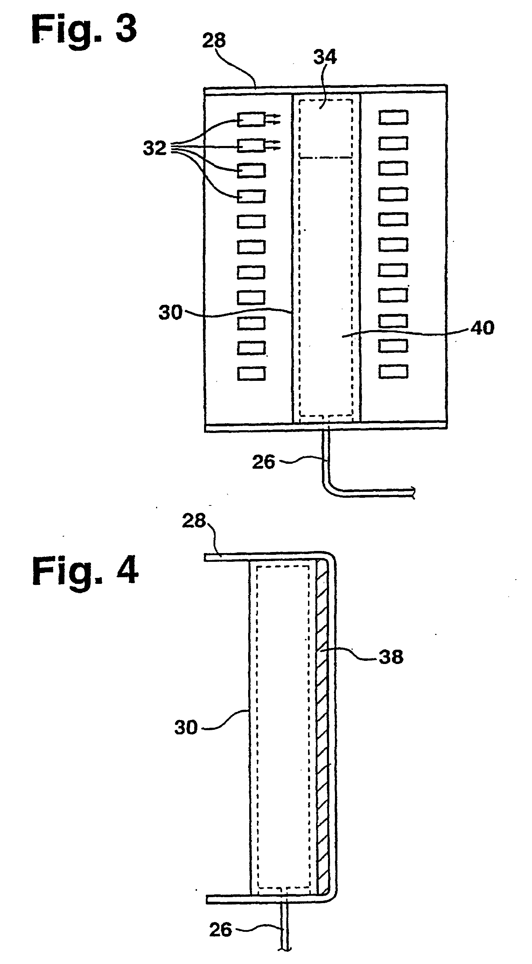

In den Figuren 3 und 4 ist die Meßvorrichtung 2 geschnitten dargestellt. Hieraus erkennt man, daß in einem metallenen Gehäuse 28 ein vertikal angeordnetes, als Glasrohr ausgeführter Meßbehälter 30 integriert ist. In diesen Meßbehälter 30 mündet die Dampfzuführleitung 26, deren Innendurchmesser deutlich kleiner ist, als derjenige des Meßbehälters 30. Der Meßbehälter 30 ist bis auf die kleine Öffnung an der Unterseite gasdicht geschlossen. Diese Öffnung des Meßbehälters 30 ist so gewählt, daß das Kondensat aufgrund der Kapillarität nicht herausfließen kann. In der hier dargestellten Ausführungsform der erfindungsgemäßen Meßvorrichtung beträgt der Durchmesser der Öffnung des Meßbehälters 30 zirka 2 mm. Auch die Dampfzuführleitung 26 hat einen Innendurchmesser von 2 mm. Hierdurch wird erreicht, daß das durch im Meßbehälter 30 auskondensierendes Wasser gebildete Kondensat aufgrund der Oberflächenspannung des Wassers nicht aus dem Meßbehälter 30 herausfließt, sondern im selbigen verbleibt.

In der hier dargestellten Ausführungsform ist der Meßbehälter 30 als Glasrohr ausgeführt, in dessen oberen Bereich mehrere Lichtschranken 32 jeweils übereinanderliegend angeordnet sind. Diese Lichtschranken 32 sprechen immer dann an, wenn sich an der betreffenden Stelle eine Gasblase 34 ausgebildet hat.

Damit der in dem Meßbehälter 30 gelangende Sattdampf auskondensiert, ist es notwendig, die Meßvorrichtung 2 zu kühlen und die beim Kondensieren anfallende Wärme abzuführen. Zu diesem Zweck ist das Gehäuse 28 aus einem gut leitenden Metall gefertigt. Außerdem sind die Hohlräume zwischen dem Meßbehälter 30 und dem Gehäuse 28 mit einer Wärmeleitpaste 38 ausgefüllt, um einen guten Abtransport der anfallenden Wärme zu gewährleisten.

Die in den Figuren 2 bis 4 dargestellte Meßvorrichtung 2 kann über die Dampfzuführleitung 26 wahlweise an die Druckkammer 4 oder an die Sattdampfzuführleitung 20 angeschlossen werden. Dabei hat ein Anschluß an die Druckkammer 4 den Vorteil, daß auch etwaige Leckagen der Druckkammer 4 durch die Meßvorrichtung 2 erkannt werden können.

Nachfolgend wird ein Beispiel eines erfindungsgemäßen Verfahrens erläutert:

Der kommunizierend mit der Druckkammer 4 verbundene Meßbehälter 30 wird während der Entlüftungsphase 6 zusammen mit der Druckkamer 4 mehrfach evakuiert, so daß am Ende der Entlüftungsphase 6 in beiden ein Vakuum anliegt. Da am Ende der Entlüftungsphase 6 der Unterdruck am größten ist, besteht zu diesem Zeitpunkt die größte Gefahr, daß über eine etwaig vorhandene Leckage Umgebungsluft in die Druckkammer eindringt.

In der anschließenden Steigephase 8 wird die Druckkammer 4 mit Sattdampf befüllt. Dieser Sattdampf verteilt sich in der gesamten Druckkammer 4 und gelangt folglich auch über die Dampfzuführleitung 26 in den Meßbehälter 30. Da der Meßbehälter 30 im Gegensatz zur Druckkammer 4 eine sehr viel niedrigere Temperatur aufweist, kondensiert der im Meßbehälter 30 befindliche Sattdampf sofort aus. Die dabei entstehende Wärme wird nach aussen abgeführt. Das hierbei entstehende Kondensat 40 sammelt sich aufgrund der Schwerkraft im unteren Bereich des vertikal angeordneten, als Glasrohr ausgeführten Meßbehälters 30. Zwar kommuniziert der immer noch luftleere obere Bereich des Meßbehälters 30 nun nicht mehr direkt mit der Druckkammer 4, da das sich gebildet habende Kondensat 40 beide Bereiche voneinander trennt, dennoch herrscht in beiden Seiten derselbe Unterdruck. Mit weiterem Einströmen von Sattdampf in die Druckkammer 4 steigt der Druck in der Druckkammer 4 an und es wird weiterer Sattdampf über die Dampfzuführleitung 26 in den Meßbehälter 30 gedrückt, der dort sofort wieder kondensiert.

Da die Öffnung des Meßbehälters 30 nur zirka 2 mm beträgt, kann das Kondensat 40 aufgrund der Kapillarität nicht in die Druckkammer 4 zurückfließen.

Beim Kondensieren des Sattdampfes im Meßbehälter 30 bildet das im Sattdampf befindliche Wasser das Kondensat, während die unerwünschten Inertgase nicht kondensieren, sondern im gasförmigen Zustand verbleiben. Da das spezifische Gewicht der Inertgase kleiner ist als das des Kondensats, steigen die Inertgase nach oben und sammeln sich im oberen Bereich des gasdichten Meßbehälters 30.

Dieser Prozeß setzt sich solange fort, bis in der Druckkammer 4 der Sterilisationsdruck anliegt. Dabei gelangt solange Sattdampf in den Meßbehälter 30, bis der sich im oberen Bereich des Meßbehälters ausbildende Druck demjenigen in der Druckkammer 4 entspricht. Der Meßbehälter 30 ist so dimensioniert, daß er gegen Ende des Sterilisationsprozesses gerade eben vollständig mit Kondensat 40 gefüllt wäre, wenn sich keinerlei Inertgase im Sattdampf befinden würden.

Die sich im oberen Bereich des Meßbehälters sammelnden Inertgase bilden dort eine Gasblase 34.

Gegen Ende des Sterilisationsprozesses werden nun die am Meßbehälter 30 angebrachten Lichtschranken 32 aktiviert und es wird festgestellt, wie viele dieser Lichtschranken ansprechen. Über die Anzahl der ansprechenden Lichtschranken 32 wird dann die ungefähre Größe der Gasblase 34 bestimmt und über Vergleichswerte festgestellt, wie groß der Inertgasanteil am Sattdampf während dieses einen Sterilisationsprozesses war. Bewegt sich der Inertgasanteil in einem zulässigen Bereich, so wird am Ende des Sterilisationsprozesses eine entsprechende Nachricht ausgegeben. Überschreitet der Inertgasanteil den zulässigen Grenzwert, so wird ebenfalls eine entsprechende Nachricht ausgegeben und der Sterilisationsprozeß muß wiederholt werden. Außerdem muß in diesem Falle überprüft werden, ob die Sterilisationsanlage noch funktionstüchtig ist oder ob sie ein Leck aufweist.

In einer anderen, nicht dargestellten Ausführungsform ist am Meßbehälter 30 lediglich eine einzige Lichtschranke angebracht. Diese einzige Lichtschranke 32 ist so positioniert, daß sie erst bei einem unzulässig hohen Inertgasanteil anspricht. In diesem Fall kann auf die anderen Lichtschranken verzichtet werden, was zu einer weiteren Kostenersparnis führt.

Das erfindungsgemäße Verfahren und die erfindungsgemäße Vorrichtung haben weiterhin den Vorteil, daß sie den Sattdampf während der Steigphase 8 überprüfen. Zu Beginn der Steigphase ist der Unterdruck in der Druckkammer 4 am größten, so daß bei einem etwaig vorhandenen Leck in der Druckkammer 4 in dieser Phase am meisten Umgebungsluft in die Druckkammer 4 eindringt. Diese gelangt anteilig in den Meßbehälter und wird erfaßt.

Nach Beendigung des Sterilisationssprozesses wird die Druckkammer 4 wieder evakuiert. Hierbei wird auch der Meßbehälter 30 entleert, da der in der Druckkammer 4 anliegende Unterdruck das Kondensat 40 aus dem Meßbehälter 30 heraussaugt. Somit wird der Meßbehälter 30 für den nächsten Sterilisationsprozeß vorbereitet und kann mit der nächsten Charge sofort wieder in Aktion treten. Somit liefert die erfindungsgemäße Vorrichtung 2, ohne zwischendurch gewartet, bedient oder zurückgestellt zu werden, sofort wieder zuverlässige Meßergebnisse.

### Bezugszeichenliste:

- 2: Meßvorrichtung
- 4: Druckkammer
- 6: Entlüftungsphase
- 8: Steigphase
- 10: Plateauphase
- 12: Trockenphase
- 14: Belüftungsphase
- 18: Dampfventil
- 20: Sattdampfzuführungsleitung
- 22: Vakuumventil
- 24: Evakuierungsleitung
- 26: Dampfzuführleitung
- 28: Gehäuse
- 30: Meßbehälter
- 32: Lichtschranke
- 34: Gasblase
- 38: Wärmeleitpaste
- 40: Kondensat

## Patentansprüche

1. Verfahren zur Analyse von Dampf aus einer Dampfsterilisations- oder Dampfdesinfektionsanlage, wobei der zu analysierende Dampf einer Druckkammer (4) der Dampfsterilisations- oder Dampfdesinfektionsanlage entnommen wird, und über eine Dampfzuführleitung (26) in einen in seinem oberen Bereich gasdicht ausgebildeten Meßbehälter (30) einer Meßvorrichtung (2) geleitet wird, wobei der im Meßbehälter (30) befindliche Dampf abgekühlt wird, so dass das im Dampf enthaltene Wasser kondensiert, wobei die im Dampf enthaltenen-Gase nach oben steigen und im oberen Bereich des Meßbehälters (30) eine Gasblase (34) bilden, und wobei die Größe der im Meßbehälter (30) befindlichen Gasblase (34) gemessen wird,
**dadurch gekennzeichnet,**
**dass** der Meßbehälter (30) kommunizierend mit der Druckkammer (4) verbunden ist, und dass die während des Sterilisations- oder Desinfektionsvorganges im Meßbehälter (30) entstandene Gasblase (34) optoelektronisch vermessen wird, wobei das Kondensat (40) aufgrund von Kapillarität im Meßbehälter (30) gehalten wird.

2. Verfahren nach Anspruch 1,
**dadurch gekennzeichnet,**
**daß** die Größe der Gasblase (34) mittels mehrerer, untereinander angeordneter Lichtschranken (32) gemessen wird, wobei die Anzahl der ansprechenden Lichtschranken (32) ein Maß für die Größe der Gasblase (34) ist.

3. Verfahren nach Anspruch 1,
**dadurch gekennzeichnet,**
**daß** die Größe der Gasblase mittels einer Lichtschranke gemessen wird, wobei die Lichtschranke anspricht, falls die Gasblase eine vorbestimmte Größe erreicht hat.

4. Vorrichtung zur Analyse von Dampf aus einer Dampfsterilisationsoder Dampfdesinfektionsanlage, mit einer kühlbaren Meßvorrichtung (2) mit einem im oberen Bereich gasdicht ausgebildeten Meßbehälter (30), mit mindestens einem im oberen Bereich des Meßbehälters (30) angeordneten Meßgerät zum Messen der Größe einer Gasblase (34) und mit einer Dampfzuführleitung (26), die an eine Druckkammer (4) der Dampfsterilisations- oder Dampfdesinfektionsanlage anbringbar ist, und in den unter die Kondensationstemperatur abkühlbaren Meßbehälter (30) mündet,
**dadurch gekennzeichnet,**
**dass** der Meßbehälter (30) kommunizierend mit der Druckkammer (4) verbunden ist, und dass das Meßgerät als optoelektronisches Meßgerät ausgeführt ist, wobei der Durchmesser einer Öffnung des Meßbehälters (30) für die Dampfzuführleitung (26) so gewählt ist, daß das Kondensat (40) aufgrund von Kapillarität nicht herausfließt.

5. Vorrichtung nach Anspruch 4,
**dadurch gekennzeichnet,**
**daß** der Meßbehälter (30) als ein einseitig verschlossenes Glasrohr ausgeführt ist.

6. Vorrichtung nach einem der Ansprüche 4 oder 5,
**dadurch gekennzeichnet,**
**daß** der Durchmesser der Öffnung des Meßbehälters (30) kleiner als 3 mm ist.

7. Vorrichtung nach einem der Ansprüche 4 bis 6,
**dadurch gekennzeichnet,**
**daß** die Dampfzuführleitung (26) als Kapillarleitung ausgeführt ist, insbesondere daß der lichte Innendurchmesser der Dampfzuführleitung (26) kleiner als 3 mm ist.

8. Vorrichtung nach einem der Ansprüche 4 bis 7,
**dadurch gekennzeichnet,**
**daß** ein einziges optoelektrisches Meßgerät vorhanden ist und daß das optoelektronische Meßgerät als Lichtschranke (32) ausgeführt ist.

9. Vorrichtung nach Anspruch 8,
**dadurch gekennzeichnet,**
**daß** die Lichtschranke (32) im oberen Bereich des Meßgerätes derart angeordnet ist, daß sie anspricht, falls die Gasblase eine vorbestimmte Größe erreicht hat.

10. Vorrichtung nach einem der Ansprüche 4 bis 9,
**dadurch gekennzeichnet,**
**daß** mehrere optoelektrische Meßgeräte als Lichtschranken (32) ausgebildet sind, daß die Lichtschranken (32) im oberen Bereich des Meßbehäiters (30) untereinander angeordnet sind, so daß die Anzahl der ansprechenden Lichtschranken (32) ein Maß für die Größe der Gasblase (34) ist.

## Claims

1. Method of analysing the steam of a steam sterilisation or steam disinfection facility, the steam to be analysed being tapped from a pressure chamber (4) of the steam sterilisation or steam disinfection facility and being directed through a steam feed line (26) to a measuring tank (30) of a measuring device (2) that is configured to be gas-tight in the upper portion thereof, the steam contained in the measuring tank (30) being cooled so that the water contained in the steam condenses, the gases contained in the steam ascending and forming a gas bubble (34) in the upper portion of the measuring tank (30), and the size of the gas bubble (34) inside the measuring tank (30) being measured,
**characterized in that**
the measuring tank (30) is connected for communication to the pressure chamber (4) and that the gas bubble (34) formed in the measuring tank (30) during the sterilisation or disinfection process is measured optoelectronically, whereas the condensate (40) being kept within the measuring tank (30) due to capillarity.

2. The method according to claim 1,
**characterized in that**
the size of the gas bubble (34) is measured by means of several photosensitive devices (32) arranged one beneath the other, the number of responsive photosensitive devices (32) being indicative of the size of the gas bubble (34).

3. The method according to claim 1,
**characterized in that**
the size of the gas bubble is measured by means of a photosensitive device, said photosensitive device being responsive if the gas bubble has reached a predetermined size.

4. A device for analysing the steam of a steam sterilisation or steam disinfection facility with a coolable measuring device (2) having a measuring tank (30) configured to be gas-tight in the upper portion thereof, with at least one measuring apparatus disposed in the upper portion of the measuring tank (30) for measuring the size of a gas bubble (34) and with a steam feed line (26) that is mountable on a pressure chamber (4) of the steam sterilisation or steam disinfection facility and discharges into the measuring tank (30) which is coolable to a temperature below the condensation temperature,
**characterized in that**
the measuring tank (30) is connected for communication to the pressure chamber (4) and that the measuring apparatus is configured as an optoelectronic measuring apparatus, the diameter of an aperture of the measuring tank (30) for the steam feed line (26) being chosen such that the condensate (40) does not flow therefrom due to capillarity.

5. The device according to claim 4,
**characterized in that**
the measuring tank (30) is configured as a glass tube that is closed on one side.

6. The device according to one of the claims 4 or 5,
**characterized in that**
the diameter of the aperture of the measuring tank (30) is smaller than 3 mm.

7. The device according to one of the claims 4 through 6,
**characterized in that**
the steam feed line (26) is configured as a capillary line, more specifically that the inside diameter of the steam feed line (26) is smaller than 3 mm.

8. The device according to one of the claims 4 through 7,
**characterized in that**
there is provided but one single opto-electrical measuring apparatus and that said opto-electronical measuring apparatus is configured as a photosensitive device (32).

9. The device according to claim 8,
**characterized in that**
the photosensitive device (32) is disposed in the upper portion of the measuring apparatus in such a manner that it is responsive when the gas bubble has reached a predetermined size.

10. The device according to one of the claims 4 through 9,
**characterized in that**
several opto-electrical measuring devices are configured as photosensitive devices (32), that the photosensitive devices (32) are disposed one beneath the other in the upper portion of the measuring tank (30) so that the number of responsive photosensitive devices (32) is indicative of the size of the gas bubble (34).

## Revendications

1. Procédé destiné à l'analyse de la vapeur dans un stérilisateur ou un désinfecteur à vapeur, la vapeur à analyser étant prélevée d'une chambre sous pression (4) d'un stérilisateur ou d'un désinfecteur et étant acheminée via une conduite d'amenée de vapeur (26) vers une enceinte de mesure (30) d'un dispositif de mesure (2) réalisée de manière à être étanche aux gaz dans sa partie supérieure, la vapeur contenue dans l'enceinte de mesure (30) étant refroidie pour que l'eau présente dans la vapeur se condense, les gaz présents dans la vapeur remontant et formant une bulle de gaz (34) dans la partie supérieure de l'enceinte de mesure (30), la taille de la bulle de gaz (34) qui se trouve dans l'enceinte de mesure (30) étant mesurée,
**caractérisé en ce que**
l'enceinte de mesure (30) est reliée à la chambre de mesure (4) de manière à communiquer avec elle et que la bulle de gaz (34) qui s'est formée dans l'enceinte de mesure (30) pendant le processus de stérilisation ou de désinfection est mesurée optoélectroniquement, le condensat (40) étant maintenu dans l'enceinte de mesure (30) en raison de la capillarité.

2. Procédé selon la revendication 1,
**caractérisé en ce que**
la taille de la bulle de gaz (34) est mesurée au moyen de plusieurs barrières photoélectriques (32) disposées les unes en dessous des autres, le nombre de barrières photoélectriques (32) réagissant permettant d'évaluer la taille de la bulle de gaz (34).

3. Procédé selon la revendication 1,
**caractérisé en ce que**
la taille de la bulle de gaz est mesurée au moyen d'une barrière photoélectrique, ladite barrière photoélectrique réagissant lorsque la bulle de gaz a atteint une taille prédéterminée.

4. Dispositif destiné à l'analyse de la vapeur dans un stérilisateur ou un désinfecteur à vapeur, avec un dispositif de mesure (2) apte à être refroidi avec une enceinte de mesure (30) réalisée de manière à être étanche aux gaz dans sa partie supérieure, avec au moins un appareil de mesure destiné à mesurer la taille d'une bulle de gaz (34) disposé dans la partie supérieure de l'enceinte de mesure (30) et avec une conduite d'amenée de vapeur (26) qui est apte à être montée sur une chambre sous pression (4) du stérilisateur ou du désinfecteur et qui débouche dans l'enceinte de mesure (30) apte à être refroidie en dessous de la température de condensation,
**caractérisé en ce que**
l'enceinte de mesure (30) est reliée à la chambre sous pression (4) de manière à communiquer avec elle et que l'appareil de mesure est réalisé sous forme d'appareil de mesure optoélectronique, le diamètre d'une ouverture ménagée dans l'enceinte de mesure (30) et destinée à la conduite d'amenée de vapeur (26) étant choisi de manière à empêcher le condensat (40) de s'en écouler en raison de la capillarité.

5. Dispositif selon la revendication 4,
**caractérisé en ce que**
l'enceinte de mesure (30) est réalisée sous forme de tube de verre fermé sur un côté.

6. Dispositif selon l'une des revendications 4 ou 5,
**caractérisé en ce que**
le diamètre de l'ouverture de l'enceinte de mesure (30) est inférieur à 3 mm.

7. Dispositif selon l'une quelconque des revendications 4 à 6,
**caractérisé en ce que**
la conduite d'amenée de vapeur (26) est réalisée sous forme de tube capillaire, notamment que le diamètre interne de la conduite d'amenée de vapeur (26) est inférieur à 3 mm.

8. Dispositif selon l'une quelconque des revendications 4 à 7,
**caractérisé en ce qu'**
un seul appareil de mesure optoélectrique est prévu et que l'appareil de mesure optoélectronique est réalisé sous forme de barrière photoélectrique (32).

9. Dispositif selon la revendication 8,
**caractérisé en ce que**
la barrière photoélectrique (32) est disposée dans la partie supérieure de l'appareil de mesure de telle sorte que celle-ci réagit lorsque la bulle de gaz a atteint une taille prédéterminée.

10. Dispositif selon l'une quelconque des revendications 4 à 9,
**caractérisé en ce que**
plusieurs appareils optoélectriques sont réalisés sous forme de barrières photoélectriques (32), que les barrières photoélectriques (32) sont disposées l'une en dessous de l'autre dans la partie supérieure de l'enceinte de mesure (30), de sorte que le nombre de barrières photoélectriques (32) réagissant permet d'évaluer la taille de la bulle de gaz (34).
